(19) Europäisches Patentamt
European Patent Office
Office européen des brevets

(11) **EP 1 517 131 A1**

(12) **EUROPÄISCHE PATENTANMELDUNG**

(43) Veröffentlichungstag:
23.03.2005 Patentblatt 2005/12

(51) Int Cl.⁷: **G01N 15/08**

(21) Anmeldenummer: 04104209.4

(22) Anmeldetag: 02.09.2004

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **19.09.2003 DE 10343516**

(71) Anmelder: **Voith Paper Patent GmbH**
**89522 Heidenheim (DE)**

(72) Erfinder:
• **Ischdonat, Thomas**
**89429 Bachhagel (DE)**
• **Pfifferling, Ralf**
**89547 Gerstetten (DE)**
• **Sollinger, Michael**
**70184 Stuttgart (DE)**
• **Muench, Rudolf**
**89551 Koenigsbronn (DE)**

(54) **Verfahren und Vorrichtung zur bestimmung wenigstend einer Eigenschaft einer bewegten Bespannung**

(57) Ein Verfahren zur Bestimmung wenigstens einer Eigenschaft einer bewegten Bespannung einer Maschine zur Herstellung und/oder Behandlung einer Materialbahn, insbesondere Papier- oder Kartonbahn, umfasst die folgenden Schritte:

Anordnen einer Düse gegen die Oberfläche der bewegten Bespannung, Erzeugen eines die Bespannung beaufschlagenden Strahles mittels der die bewegte Bespannung berührenden Düse,
Messung einer für den Durchfluss durch die Düse repräsentativen Größe, Messung einer für den Druck des Strahles am Düsenaustritt repräsentativen Größe,
Bestimmung der Geschwindigkeit des Strahles am Düsenaustritt aus dem Düsendurchfluss und der freien Oberfläche des Strahlmediums an der Berührungsstelle mit der Bespannung und
Bestimmung der Geschwindigkeit des Strahles am Eintritt in die Bespannung aus der Geschwindigkeit des Strahles am Düsenaustritt und der Geschwindigkeit der Bespannung,

wobei anhand der Geschwindigkeit des Strahles am Eintritt in die Bespannung bei einem gegebenen Druck des Strahles am Düsenaustritt bzw. Eintritt in die Bespannung die Wasserpermeabilität der bewegten Bespannung bestimmt und/oder wenigstens eine Oberflächeneigenschaft der bewegten Bespannung bestimmt wird. Es wird auch eine entsprechende Vorrichtung angegeben.

Fig.1

EP 1 517 131 A1

**Beschreibung**

Die Erfindung betrifft ein Verfahren sowie eine Vorrichtung zur Bestimmung wenigstens einer Eigenschaft einer bewegten Bespannung einer Maschine zur Herstellung und/oder Behandlung einer Materialbahn, insbesondere Papier- oder Kartonbahn.

Verfahren und Vorrichtungen zur Bestimmung der Wasserpermeabilität von wasserdurchlässigen Bespannungen oder Geweben in einer Papiermaschine mittels einer gegen die Oberfläche der Bespannung gerichteten Düsenvorrichtung sind beispielsweise aus den Druckschriften DE 199 17 553 A1 und US 4 880 499 bekannt. Gemäß der DE 199 17 553 A1 wird unter Aufrechterhaltung einer konstanten Wasserströmung der Druck in der Düse gemessen, wobei die Wasserströmung wahlweise mit einem Gas, insbesondere Luft, beaufschlagt wird. Dagegen wird in der US 4 880 499 vorgeschlagen, den Durchfluss durch die Düsenvorrichtung unter Aufrechterhaltung eines konstanten Drucks in einem Bereich von 2 bis 1000 kPa innerhalb der Düsenvorrichtung zu messen. Der Druck bzw. der Durchfluss werden dann als zur Permeabilität proportionale Messgröße herangezogen.

Beide Verfahren bzw. Vorrichtungen sind zwar geeignet, die Permeabilität von wasserdurchlässigen Geweben im stationären Zustand zu bestimmen. In der Praxis werden die Gewebe üblicherweise jedoch mit Geschwindigkeiten bis zu 2200 m/min bewegt. Wird die Wasserpermeabilität bei einem der bekannten Verfahren unter den soeben genannten Bedingungen gemessen, so führt dies zu einem stark verfälschten Messergebnis, das nur noch wenige Rückschlüsse auf die wirkliche Permeabilität zulässt. Eine vergleichende Messung bei unterschiedlichen Gewebegeschwindigkeiten ist kaum möglich.

Der Erfindung liegt die Aufgabe zugrunde, ein verbessertes Verfahren sowie eine verbesserte Vorrichtung der eingangs genannten Art zu schaffen, bei der die zuvor genannten Nachteile beseitigt sind. Dabei soll insbesondere eine von der Geschwindigkeit der Bespannung relativ zur Messvorrichtung bzw. Düse unabhängige Messung der Wasserpermeabilität der wasserdurchlässigen Bespannung möglich sein.

Diese Aufgabe wird erfindungsgemäß gelöst durch ein Verfahren zur Bestimmung wenigstens einer Eigenschaft einer bewegten Bespannung einer Maschine zur Herstellung und/oder Behandlung einer Materialbahn, insbesondere Papier- oder Kartonbahn, mit den folgenden Schritten:

Anordnen einer Düse gegen die Oberfläche der bewegten Bespannung, Erzeugen eines die Bespannung beaufschlagenden Strahles mittels der die bewegte Bespannung berührenden Düse,
Messung einer für den Durchfluss durch die Düse repräsentativen Größe, Messung einer für den Druck des Strahles am Düsenaustritt repräsentativen Größe,
Bestimmung der Geschwindigkeit des Strahles am Düsenaustritt aus dem Düsendurchfluss und der freien Oberfläche des Strahlmediums an der Berührungsstelle mit der Bespannung und
Bestimmung der Geschwindigkeit des Strahles am Eintritt in die Bespannung aus der Geschwindigkeit des Strahles am Düsenaustritt und der Geschwindigkeit der Bespannung,

wobei anhand der Geschwindigkeit des Strahles am Eintritt in die Bespannung bei einem gegebenen Druck des Strahles am Düsenaustritt bzw. Eintritt in die Bespannung die Wasserpermeabilität der bewegten Bespannung bestimmt und/oder wenigstens eine Oberflächeneigenschaft der bewegten Bespannung bestimmt wird. Bevorzugt werden nacheinander zumindest zwei verschiedene Geschwindigkeiten des Strahles am Eintritt in die Bespannung eingestellt und die gemessenen bzw. berechneten Ergebnisse miteinander verglichen.

Aufgrund dieser erfindungsgemäßen Ausgestaltung des Verfahrens ist eine von der Geschwindigkeit der Bespannung relativ zur Düse unabhängige Messung der Wasserpermeabilität des wasserdurchlässigen Gewebes möglich. Überdies können durch geeignete Variationen der Messparameter unterschiedliche Eigenschaften wie die Permeabilität und Oberflächeneigenschaften der Bespannung ermittelt werden. Die Mess- bzw. berechneten Ergebnisse ermöglichen eine gezielte Reinigung oder Konditionierung oder auch eine Lebenszeit-Analyse der Bespannung. Standzeiten der Bespannungen können auf diese Weise optimiert, und Bespannungswechsel können geplant werden, wodurch ungeplante Stillstände der Papiermaschine vermieden werden.

Bei der Bestimmung einer jeweiligen Eigenschaft der bewegten Bespannung wird zweckmäßigerweise von den jeweiligen Geschwindigkeitsvektoren ausgegangen. Dabei verlaufen die Vektoren der Geschwindigkeit des Strahles am Düsenaustritt und der Geschwindigkeit des Strahles am Eintritt in die Bespannung jeweils senkrecht zur beaufschlagten Bespannungsoberfläche bzw. senkrecht zur Geschwindigkeit der Bespannung.

Der Strahl tritt also mit einer bestimmten Geschwindigkeit am Düsenaustritt aus und trifft auf die Bespannung, wo er umgelenkt wird. Nach der Umlenkung besitzt der Strahl zwei Komponenten, von denen die eine der Geschwindigkeit des Strahls am Eintritt in die Bespannung und die andere der Geschwindigkeit der Bespannung entspricht. Die Geschwindigkeit des Strahles am Düsenaustritt ergibt sich aus dem bekannten Düsendurchfluss und der freien Oberfläche des Wassers am Berührungspunkt mit der Bespannung. Aus der Geschwindigkeit des Strahles am Düsenaustritt und der Geschwindigkeit der Bespannung kann dann über die gegebenen geometrischen Zusammenhänge, d.h. über die folgende Beziehung die Geschwindigkeit des Strahles am Eintritt in die Bespannung bestimmt werden:

$$v_e = \sqrt{v_a{}^2 - v_m{}^2},$$

mit

$v_a$= Geschwindigkeit des Strahles am Düsenaustritt

$v_e$= Geschwindigkeit des Strahles am Eintritt in die Bespannung.

$v_m$= Geschwindigkeit der Bespannung.

Der Winkel zwischen $v_e$ und $v_a$ kann zwischen 0° und 90° betragen, wobei bei einem Winkel vom 90° die Geschwindigkeit $v_m$ der Bespannung größer oder gleich der Geschwindigkeit $v_a$ des Strahles am Düsenaustritt ist. In diesem Fall nimmt die Bespannung den Strahl einfach mit. Es werden also nur Oberflächeneigenschaften der Bespannung gemessen ($v_e$ = 0).

Ist die Geschwindigkeit $v_a$ des Strahles am Düsenaustritt größer als die Geschwindigkeit $v_m$ der Bespannung, so dringt der Strahl an der Berührungsstelle mit der Eintrittsgeschwindigkeit $v_e$ in die Bespannung ein. Da sich der Strahl innerhalb der Bespannung sofort aufweitet, gilt die oben genannte geometrische Beziehung nur nahe der Oberfläche der Bespannung. Eine Eintrittsgeschwindigkeit $v_e$ liegt jedoch definitiv vor, d.h. der Strahl tritt durch die Bespannungsoberfläche hindurch.

Je größer die Eintrittsgeschwindigkeit $v_e$ im Vergleich mit der Geschwindigkeit $v_a$ des Strahles am Düsenaustritt ist, desto stärker bzw. desto tiefer wird der Strahl in die Bespannung eindringen, bevor er sich aufweitet.

Von besonderem Vorteil ist nun, wenn der Strahl in die Bespannung eindringt, bevor er sich aufweitet. Desto tiefer wird er auch eindringen.

Von besonderem Vorteil ist insbesondere auch, wenn nacheinander zumindest zwei verschiedene Geschwindigkeiten des Strahles am Düsenaustritt und/oder zumindest zwei verschiedene Geschwindigkeiten des Strahles am Eintritt in die Bespannung eingestellt und die gemessenen bzw. berechneten Ergebnisse miteinander verglichen werden.

Aus den bestimmten Werten, Ergebnissen und/oder Vergleichen kann insbesondere auf zumindest eine Permeabilitäts- und/oder Oberflächeneigenschaft der Bespannung geschlossen werden.

Für Messungen mit einer Geschwindigkeit $v_e$ des Strahles am Eintritt in die Bespannung größer als Null, kann die Permeabilität bestimmt oder berechnet werden. Dabei ist die Permeabilität eine Funktion des Drucks p des Strahles am Düsenaustritt, des Durchflusses durch die Düse und der Geschwindigkeit $v_m$ der Bespannung. Je größer die Geschwindigkeit $v_a$ am Düsenaustritt ist, desto größer ist auch die Tiefenwirkung der Permeabilitätsmessung. Es lassen sich also Permeabilitäten bei verschiedenen Tiefenwirkungen ermitteln. Ist bei einer Geschwindigkeit $v_e$ des Strahles am Eintritt in die Bespannung gleich Null, werden dagegen nur Oberflächeneigenschaften der Bespannung erfasst.

Praktisch dringt auch bei einer Geschwindigkeit $v_e$ des Strahles am Eintritt in die Bespannung gleich Null der Strahl geringfügig in die Bespannung ein. Dabei entspricht die Eindringtiefe maximale der Dicke des ursprünglichen Strahls. Angesichts der unvermeidlichen Aufweitung ist die tatsächliche Eindringtiefe geringer. Zudem ist zu berücksichtigen, dass die Oberfläche der Bespannung rau ist und eine gewisse Menge Wasser mitschleppen kann, ohne dass ein Eindringen erfolgt. Wird die Geschwindigkeit $v_a$ des Strahles am Düsenaustritt noch kleiner, gilt also $v_a < v_m$, so verringert sich die Möglichkeit eines begrenzten Eindringens des Strahles weiter.

Daraus folgt überdies, dass für eine Anwendung der Erfindung an schnelllaufenden Papiermaschinen Drücke erforderlich sind, die weit größer als 6 bar sind. Bei 6 bar (60 m Wassersäule) ist die Geschwindigkeit $v_a$ des Strahles am Düsenaustritt erst $\sqrt{2 * g * h}$ = 2100 m/min, was immer noch in der Größenordnung der Geschwindigkeit $v_m$ der Bespannung liegt.

Gemäß einer bevorzugten praktischen Ausgestaltung des erfindungsgemäßen Verfahrens wird der Druck des Strahles am Düsenaustritt also größer als 6 bar gewählt.

Ein weiterer Ansatz, die Tiefenwirkung separat zu erfassen, besteht darin, zumindest zwei sich bei unterschiedlichen Drücken p des Strahles am Düsenaustritt ergebende Arbeitspunkte miteinander zu vergleichen. Dabei werden die unterschiedlichen Drücke des Strahles am Düsenaustritt vorzugsweise jeweils größer als 6 bar gewählt. Dabei kann beispielsweise für einen Arbeitspunkt etwa 10 und für den anderen Arbeitspunkt etwa 20 bar gewählt werden.

Über die betreffenden geometrischen Beziehungen erhält man dann wieder:

$$v_{e1} = \sqrt{v_{a1}{}^2 - v_m{}^2}$$

$$v_{e2} = \sqrt{v_{a2}{}^2 - v_m{}^2}$$

$$\Delta v_e = v_{e2} - v_{e1}$$

$$\Delta p = 20 \text{ bar} - 10 \text{ bar} = 10 \text{ bar}$$

$$\text{Tiefenpermeabilität} = \Delta v_e / \Delta p$$

Dadurch wird auch die Verfälschung des Messergebnisses durch Oberflächeneffekte verringert, die bei geringeren Drücken noch dominierend sein können.

Die bewegte Bespannung kann insbesondere durch ein Gewebeband, beispielsweise durch einen Filz gebildet sein.

Das Strahlmedium enthält bevorzugt Wasser.

Die eingangs angegebene Aufgabe wird erfindungsgemäß überdies gelöst durch eine Vorrichtung zur Bestimmung wenigstens einer Eigenschaft einer bewegten Bespannung einer Maschine zur Herstellung und/oder Behandlung einer Materialbahn, insbesondere Papier- oder Kartonbahn, insbesondere zur Durchführung des Verfahrens nach einem der vorhergehenden Ansprüche, mit

einer gegen die Oberfläche der bewegten Bespannung gerichteten, diese berührenden Düse zur Erzeugung eines die Bespannung beaufschlagenden Strahles,

Mitteln zur Messung einer für den Durchfluss durch die Düse repräsentativen Größe,

Mitteln zur Messung einer für den Druck des Strahles am Düsenaustritt repräsentativen Größe,

Mitteln zur Bestimmung der Geschwindigkeit des Strahles am Düsenaustritt aus dem Düsendurchfluss und der freien Oberfläche des Strahlmediums an der Berührungsstelle mit der Bespannung,

Mitteln zur Bestimmung der Geschwindigkeit des Strahles am Eintritt in die Bespannung aus der Geschwindigkeit des Strahles am Düsenaustritt und der Geschwindigkeit der Bespannung, sowie mit

Mitteln zur Bestimmung der Wasserpermeabilität der bewegten Bespannung anhand der Geschwindigkeit des Strahles am Eintritt in die Bespannung bei einem gegebenen Druck des Strahles am Düsenaustritt bzw. Eintritt in die Bespannung und/oder zur Bestimmung wenigstens einer Oberflächeneigenschaft der bewegten Bespannung.

Bevorzugte Ausführungsformen der erfindungsgemäßen Vorrichtung sind in den Unteransprüchen angegeben.

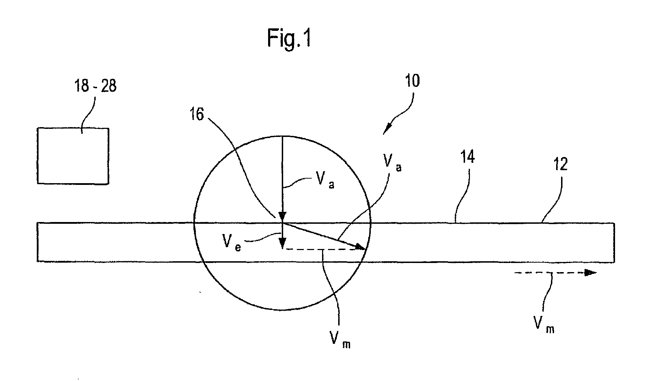

Die Erfindung wird im folgenden anhand eines Ausführungsbeispiels unter Bezugnahme auf die Zeichnung näher erläutert;

Die einzige Figur der Zeichnung zeigt in stark vereinfachter schematischer Darstellung eine Vorrichtung 10 zur Bestimmung wenigstens einer Eigenschaft einer bewegten Bespannung 12 einer Maschine zur Herstellung und/oder Behandlung einer Materialbahn, bei der es sich insbesondere um eine Papier- oder Kartonbahn handeln kann.

Die Vorrichtung 10 umfasst eine gegen die Oberfläche 14 der bewegten Bespannung 12 gerichtete, diese berührende Düse 16 zur Erzeugung eines die Bespannung 12 beaufschlagenden Strahles.

Die Vorrichtung 10 umfasst überdies hier lediglich schematisch angedeutet Mittel 18 zur Messung einer für den Durchfluss durch die Düse 16 repräsentativen Größe, Mittel 20 zur Messung einer für den Druck p des Strahles am Düsenaustritt repräsentativen Größe, Mittel 22 zur Bestimmung der Geschwindigkeit $v_a$ des Strahles am Düsenaustritt aus dem Düsenddurchfluss und der freien Oberfläche des Strahlmediums an der Berührungsstelle mit der Bespannung 12, Mittel 24 zur Bestimmung der Geschwindigkeit $v_e$ des Strahles am Eintritt in die Bespannung 12 aus der Geschwindigkeit $v_a$ des Strahles am Düsenaustritt und der Geschwindigkeit $v_m$ der Bespannung 12, sowie Mittel 26, 28 zur Bestimmung der Wasserpermeabilität der bewegten Bespannung 12 anhand der Geschwindigkeit $v_e$ des Strahles am Eintritt in die Bespannung 12 bei einem gegebenen Druck p des Strahles am Düsenaustritt bzw. Eintritt in die Bespannung 12 und/oder zur Bestimmung wenigstens einer Oberflächeneigenschaft der bewegten Bespannung 12.

Es wird also ein Messgerät mit einer Düse 16 verwendet, die berührend über die Bespannung 12 gleitet. Bei der Bespannung 12 kann es sich beispielsweise um ein Gewebeband und insbesondere um einen Filz oder dergleichen handeln. Der Durchfluss durch die Düse 16 und der Druck des Strahles, der in die Bespannung 12 eingedrückt wird, werden erfasst oder aus anderen indirekten Größen berechnet.

Ausgegangen wird von einer Definition der Permeabilität als Geschwindigkeit $v_e$, mit der der Strahl in die Bespannung bei einem gegebenen Eintrittsdruck eintritt. Das Strahlmedium kann insbesondere Wasser enthalten oder aus Wasser bestehen.

Für die Permeabilität gilt ausgehend von der angegebenen Definition also die folgende Beziehung:

$$P_{erm} = v_e/p \text{ mit}$$

$P_{erm}$ = Permeabilität
$v_e$ = Geschwindigkeit des Strahles am Eintritt in die Bespannung
p = Druck des Strahles am Düsenaustritt

Zudem wird von den jeweiligen Geschwindigkeitsvektoren ausgegangen, wobei wieder gilt:

$v_a$ = Geschwindigkeit des Strahles am Düsenaustritt
$v_e$ = Geschwindigkeit des Strahles am Eintritt in die Bespannung
$v_m$ = Geschwindigkeit der Bespannung

Der Strahl tritt also mit der Geschwindigkeit $v_a$ aus der Düse 16 aus und trifft auf die Bespannung 12 auf. Dort wird er umgelenkt. Nach der Umlenkung besitzt der Strahl zwei Komponenten, nämlich die Geschwindigkeit $v_m$ der Bespannung 12 und die Geschwindigkeit $v_e$ des Strahles am Eintritt in die Bespannung 12.

Die Geschwindigkeit $v_a$ des Strahles am Düsenaustritt ist aus dem Düsendurchfluss und der freien Oberfläche des Wassers an der Berührungsstelle mit der Bespannung 12 bekannt. Aus der Geschwindigkeit $v_a$ des Strahles am Düsenaustritt und der Geschwindig-

keit $v_m$ der Bespannung 12 kann aus der folgenden geometrischen Beziehung die Geschwindigkeit $v_e$ des Strahles am Eintritt in die Bespannung 12 bestimmt werden:

$$v_e = \sqrt{v_a^2 - v_m^2}$$

Der Winkel zwischen der Geschwindigkeit $v_e$ des Strahles am Eintritt in die Bespannung und der Geschwindigkeit $v_a$ des Strahles am Düsenaustritt kann zwischen 0° und 90° betragen. Ein Winkel von 90° ergibt sich dann, wenn die Geschwindigkeit $v_m$ der Bespannung 12 größer oder gleich der Geschwindigkeit $v_a$ des Strahles am Düsenaustritt ist. Die Bespannung nimmt dann den Strahl einfach mit. In diesem Fall werden nur Oberflächeneigenschaften der Bespannung 12 gemessen ($v_e = 0$).

Ist die Geschwindigkeit $v_a$ des Strahles am Düsenaustritt größer als die Geschwindigkeit $v_m$ der Bespannung 12, so dringt der Strahl an der Berührungsstelle mit der Eintrittsgeschwindigkeit $v_e$ in die Bespannung 12 ein. Da sich der Strahl innerhalb der Bespannung 12 sofort aufweitet, gilt die angegebene geometrische Beziehung nur nahe der Oberfläche der Bespannung 12. Eine Eindringtiefe bzw. Eintrittsgeschwindigkeit $v_e$ liegt jedoch definitiv vor. Der Strahl tritt durch die Oberfläche der Bespannung 12 hindurch.

Je größer die Geschwindigkeit $v_e$ des Strahles beim Eintritt in die Bespannung 12 im Vergleich mit der Geschwindigkeit $v_a$ des Strahles am Düsenaustritt ist, desto stärker und entsprechend tiefer wird der Strahl in die Bespannung 12 eindringen, bevor er sich aufweitet.

Von besonderem Vorteil ist nun, wenn nacheinander zwei verschiedene Düsenaustrittsgeschwindigkeiten $v_a$ und/oder zumindest zwei verschiedene Geschwindigkeiten $v_e$ des Strahles am Eintritt in die Bespannung 12 gewählt und die gemessenen bzw. berechneten Ergebnisse miteinander verglichen werden.

Für Messungen mit einer Geschwindigkeit $v_e$ des Strahles am Eintritt in die Bespannung 12 größer als Null, kann die Permeabilität berechnet werden. Dabei ist diese Permeabilität eine Funktion des Druckes p des Strahles am Düsenaustritt, des Durchflusses durch die Düse 16 und der Geschwindigkeit $v_m$ der Bespannung 12. Je größer die Geschwindigkeit $v_a$ des Strahles am Düsenaustritt ist, desto größer ist auch die Tiefenwirkung der Permeabilitätsmessung bzw. -bestimmung. Die Permeabilität lässt sich also bei verschiedenen Tiefenwirkungen ermitteln. Dagegen werden bei einer Geschwindigkeit $v_e = 0$ nur Oberflächeneigenschaften der Bespannung 12 erfasst.

Praktisch dringt auch bei einer Geschwindigkeit $v_e = 0$ des Strahles am Eintritt in die Bespannung 12 der Strahl geringfügig in die Bespannung 12 ein. Dabei entspricht die Eindringtiefe maximal der Dicke des ursprünglichen Strahls. Angesichts der unvermeintlichen Aufweitung ist die tatsächliche Eindringtiefe jedoch geringer. Zudem ist zu berücksichtigen, dass die Oberfläche der Bespannung 12 rau ist und auch eine gewisse Menge Wasser mitschleppen kann, ohne dass ein Eindringen erfolgt. Wird die Strahlgeschwindigkeit noch kleiner, gilt also $v_a < v_m$, so verringert sich die Möglichkeit eines begrenzten Eindringens des Strahles weiter.

Daraus folgt auch, dass für die Anwendung der erfindungsgemäßen Lösung an schnelllaufenden Papiermaschinen Drücke zweckmäßig sind, die weit größer als 6 bar sind. Bei 6 bar (60 m Wassersäule) beträgt die Geschwindigkeit $v_a$ des Strahles am Düsenaustritt erst $\sqrt{2 \ast g \ast h}$ = 2100 m/min, was immer noch in der Größenordnung der Geschwindigkeit $v_m$ der Bespannung 12 liegt.

Ein weiterer Ansatz, die Tiefenwirkung getrennt zu erfassen, besteht darin, zumindest zwei Arbeitspunkte des Messgerätes bzw. der Vorrichtung 10 bei relativ hohen Drücken miteinander zu vergleichen. So kann beispielsweise für einen Arbeitspunkt 10 bar und für den anderen Arbeitspunkt 20 bar gewählt werden. Über die geometrische Beziehung erhält man dann:

$$v_{e1} = \sqrt{(v_{a1}^2 - v_m^2)},$$

$$v_{e2} = \sqrt{(v_a^2 - v_m^2)}$$

$$\Delta v_e = v_{e2} - v_{e1}$$

$$\Delta p = 20 \text{ bar} - 10 \text{ bar} = 10 \text{ bar}$$

$$\text{Tiefenpermeabilität} = \Delta v_e / \Delta p.$$

Damit wird entsprechend auch die Verfälschung des Messergebnisses durch Oberflächeneffekte verringert, die bei geringen Drücken noch dominierend sein können.

**Bezugszeichenliste**

| | |
|---|---|
| 10 | Vorrichtung |
| 12 | Bespannung, Gewebeband, Filz |
| 14 | Oberfläche der Bespannung |
| 16 | Düse |
| 18 | Mittel zur Durchflussmessung |
| 20 | Mittel zur Druckmessung |
| 22 | Mittel zur Bestimmung der Strahlaustrittsgeschwindigkeit |
| 24 | Mittel zur Bestimmung der Strahleintrittsgeschwindigkeit |

26 Mittel zur Bestimmung der Wasserpermeabilität
28 Mittel zur Bestimmung wenigstens einer Oberflächeneigenschaft

**Patentansprüche**

1. Verfahren zur Bestimmung wenigstens einer Eigenschaft einer bewegten Bespannung (12) einer Maschine zur Herstellung und/oder Behandlung einer Materialbahn, insbesondere Papieroder Kartonbahn, mit den folgenden Schritten:

   Anordnen einer Düse (16) gegen die Oberfläche (14) der bewegten Bespannung (12),
   Erzeugen eines die Bespannung (12) beaufschlagenden Strahles mittels der die bewegte Bespannung (12) berührenden Düse (16),
   Messung einer für den Durchfluss durch die Düse (16) repräsentativen Größe,
   Messung einer für den Druck (p) des Strahles am Düsenaustritt repräsentativen Größe,
   Bestimmung der Geschwindigkeit ($v_a$) des Strahles am Düsenaustritt aus dem Düsendurchfluss und der freien Oberfläche des Strahlmediums an der Berührungsstelle mit der Bespannung (12) und
   Bestimmung der Geschwindigkeit ($v_e$) des Strahles am Eintritt in die Bespannung (12) aus der Geschwindigkeit ($v_a$) des Strahles am Düsenaustritt und der Geschwindigkeit ($v_m$) der Bespannung (12), wobei anhand der Geschwindigkeit ($v_e$) des Strahles am Eintritt in die Bespannung (12) bei einem gegebenen Druck (p) des Strahles am Düsenaustritt bzw. Eintritt in die Bespannung (12) die Wasserpermeabilität der bewegten Bespannung (12) bestimmt und/oder wenigstens eine Oberflächeneigenschaft der bewegten Bespannung (12) bestimmt wird.

2. Verfahren nach Anspruch 1,
   **dadurch gekennzeichnet,**
   **dass** nacheinander zumindest zwei verschiedene Geschwindigkeiten ($v_a$) des Strahles am Eintritt in die Bespannung (12) eingestellt und die gemessenen bzw. berechneten Ergebnisse miteinander verglichen werden.

3. Verfahren nach Anspruch 1 oder 2,
   **dadurch gekennzeichnet**,
   aus den bestimmten Werten, Ergebnissen und/oder Vergleichen auf zumindest eine Permeabilitätsund/oder Oberflächeneigenschaft der Bespannung (12) geschlossen wird.

4. Verfahren nach einem der vorhergehenden Ansprüche,

   **dadurch gekennzeichnet,**
   **dass** bei der Bestimmung einer jeweiligen Eigenschaft der bewegten Bespannung (12) von den jeweiligen Geschwindigkeitsvektoren ausgegangen.

5. Verfahren nach Anspruch 4,
   **dadurch gekennzeichnet,**
   **dass** die Vektoren der Geschwindigkeit ($v_a$) des Strahles am Düsenaustritt und der Geschwindigkeit ($v_e$) des Strahles am Eintritt in die Bespannung (12) jeweils senkrecht zur beaufschlagten Bespannungsoberfläche (14) sowie senkrecht zur Geschwindigkeit (vm) der Bespannung (12) verlaufen.

6. Verfahren nach einem der vorhergehenden Ansprüche,
   **dadurch gekennzeichnet,**
   **dass** der Druck (p) des Strahles am Düsenaustritt größer als 6 bar gewählt wird.

7. Verfahren nach einem der vorhergehenden Ansprüche,
   **dadurch gekennzeichnet,**
   **dass** zumindest zwei sich bei unterschiedlichen Drücken (p) des Strahles am Düsenaustritt ergebende Arbeitspunkte miteinander verglichen werden.

8. Verfahren nach Anspruch 7,
   **dadurch gekennzeichnet,**
   **dass** die unterschiedlichen Drücke (p) des Strahles am Düsenaustritt jeweils größer als 6 bar gewählt werden, wobei vorzugsweise für einen Arbeitspunkt etwa 10 und für den anderen Arbeitspunkt etwa 20 bar gewählt werden.

9. Verfahren nach einem der vorhergehenden Ansprüche,
   **dadurch gekennzeichnet,**
   **dass** die bewegte Bespannung (12) durch ein Gewebeband gebildet ist.

10. Verfahren nach einem der vorhergehenden Ansprüche,
    **dadurch gekennzeichnet,**
    **dass** die bewegte Bespannung (12) durch einen Filz gebildet ist.

11. Verfahren nach einem der vorhergehenden Ansprüche,
    **dadurch gekennzeichnet,**
    **dass** das Strahlmedium Wasser enthält.

12. Vorrichtung (10) zur Bestimmung wenigstens einer Eigenschaft einer bewegten Bespannung (12) einer Maschine zur Herstellung und/oder Behandlung einer Materialbahn, insbesondere Papieroder Kartonbahn, insbesondere zur Durchführung des Verfahrens nach einem der vorhergehenden Ansprüche, mit

einer gegen die Oberfläche (14) der bewegten Bespannung (12) gerichteten, diese berührenden Düse (16) zur Erzeugung eines die Bespannung (12) beaufschlagenden Strahles,

Mitteln (18) zur Messung einer für den Durchfluss durch die Düse (16) repräsentativen Größe,

Mitteln (20) zur Messung einer für den Druck (p) des Strahles am Düsenaustritt repräsentativen Größe,

Mitteln (22) zur Bestimmung der Geschwindigkeit ($v_a$) des Strahles am Düsenaustritt aus dem Düsendurchfluss und der freien Oberfläche des Strahlmediums an der Berührungsstelle mit der Bespannung (12),

Mitteln (24) zur Bestimmung der Geschwindigkeit ($v_e$) des Strahles am Eintritt in die Bespannung (12) aus der Geschwindigkeit ($v_a$) des Strahles am Düsenaustritt und der Geschwindigkeit ($v_m$) der Bespannung (12), sowie mit

Mitteln (26, 28) zur Bestimmung der Wasserpermeabilität der bewegten Bespannung (12) anhand der Geschwindigkeit ($v_e$) des Strahles am Eintritt in die Bespannung (12) bei einem gegebenen Druck (p) des Strahles am Düsenaustritt bzw. Eintritt in die Bespannung (12) und/oder zur Bestimmung wenigstens einer Oberflächeneigenschaft der bewegten Bespannung (12).

13. Vorrichtung nach Anspruch 12,
**dadurch gekennzeichnet,**
**dass** nacheinander zumindest zwei verschiedene Geschwindigkeiten ($v_e$) des Strahles am Eintritt in die Bespannung (12) einstellbar und die gemessenen bzw. berechneten Ergebnisse miteinander vergleichbar sind.

14. Vorrichtung nach Anspruch 12 oder 13,
**dadurch gekennzeichnet,**
**dass** eine jeweilige Eigenschaft der bewegten Bespannung (12) ausgehend von den jeweiligen Geschwindigkeitsvektoren bestimmbar ist.

15. Vorrichtung nach Anspruch 14,
**dadurch gekennzeichnet,**
**dass** die Vektoren der Geschwindigkeit (va) des Strahles am Düsenaustritt und der Geschwindigkeit (ve) des Strahles am Eintritt in die Bespannung (12) jeweils senkrecht zur beaufschlagten Bespannungsoberfläche (14) sowie senkrecht zur Geschwindigkeit ($v_m$) der Bespannung (12) verlaufen.

16. Vorrichtung nach einem der vorhergehenden Ansprüche,
**dadurch gekennzeichnet,**
**dass** der Druck (p) des Strahles am Düsenaustritt größer als 6 bar gewählt ist.

17. Vorrichtung nach einem der vorhergehenden Ansprüche,
**dadurch gekennzeichnet,**
**dass** zumindest zwei sich bei unterschiedlichen Drücken (p) des Strahles am Düsenaustritt ergebenden Arbeitspunkte miteinander vergleichbar sind.

18. Vorrichtung nach Anspruch 17,
**dadurch gekennzeichnet,**
**dass** die unterschiedlichen Drücke (p) des Strahles am Düsenaustritt jeweils größer als 6 bar gewählt sind, wobei vorzugsweise für einen Arbeitspunkt etwa 10 und für einen den Arbeitspunkt etwa 20 bar gewählt sind.

19. Vorrichtung nach einem der vorhergehenden Ansprüche,
**dadurch gekennzeichnet,**
**dass** die bewegte Bespannung (12) durch ein Gewebeband gebildet ist.

20. Vorrichtung nach einem der vorhergehenden Ansprüche,
**dadurch gekennzeichnet,**
**dass** die bewegte Bespannung durch einen Filz gebildet ist.

21. Vorrichtung nach einem der vorhergehenden Ansprüche,
**dadurch gekennzeichnet,**
**dass** das Strahlmedium Wasser enthält.

Fig.1

**Europäisches Patentamt**

**EUROPÄISCHER RECHERCHENBERICHT**

Nummer der Anmeldung

EP 04 10 4209

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X,D | US 6 266 999 B1 (ARNSHAV SIVERT) 31. Juli 2001 (2001-07-31) * Zusammenfassung * * Spalte 2, Zeile 25 - Spalte 3, Zeile 17 * * Spalte 4, Zeile 64 - Spalte 5, Zeile 39 * * Abbildungen 4-6 * ----- | 1,12 | G01N15/08 |

RECHERCHIERTE SACHGEBIETE (Int.Cl.7)

G01N

Der vorliegende Recherchenbericht wurde für alle Patentansprüche erstellt

| Recherchenort | Abschlußdatum der Recherche | Prüfer |
|---|---|---|
| München | 27. Oktober 2004 | Bravin, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**                    EP 04 10 4209

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-10-2004

| Im Recherchenbericht angeführtes Patentdokument | Datum der Veröffentlichung | Mitglied(er) der Patentfamilie | Datum der Veröffentlichung |
|---|---|---|---|
| US 6266999 B1 | 31-07-2001 | SE 512011 C2<br>CA 2264346 A1<br>DE 19917553 A1<br>SE 9801434 A | 10-01-2000<br>23-10-1999<br>28-10-1999<br>24-10-1999 |

EPO FORM P0461

Für nähere Einzelheiten zu diesem Anhang : siehe Amtsblatt des Europäischen Patentamts, Nr.12/82